# EUROPEAN PATENT APPLICATION

(11) **EP 3 741 413 A1**
(43) Date of publication of application: **25.11.2020**
(21) Application number: 19176422.4
(22) Date of filing: 24.05.2019
(51) Int. Cl.: A61M 15/06, A24F 47/00, A61M 11/02, A61M 16/00

(54) **REFILL DEVICE FOR AEROSOL DELIVERY DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A refill device (901) for an aerosol delivery device. The refill device comprising: a porous liquid transfer element (902) containing aerosol precursor; and an engagement unit (903), located at one end of the refill device and including at least a portion of the porous liquid transfer element, the engagement unit being suitable for engaging with a corresponding engagement unit of the aerosol delivery device, so as to allow aerosol precursor to flow from the porous liquid transfer element into the aerosol delivery device.

## Description

### Field of the Invention

The present invention relates to a refill device for an aerosol delivery device, and particularly to a refill device for an aerosol delivery device having a porous liquid transfer element of aerosol precursor.

### Background

A smoking-substitute device is an electronic device that permits the user to simulate the act of smoking by producing an aerosol mist or vapour that is drawn into the lungs through the mouth and then exhaled. The inhaled aerosol mist or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heating device to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e liquid and is not intended to be refilled by an end user. One subset of closed system vaping smoking substitute devices include a main body which includes the power source, wherein the main body is configured to be physically and electrically coupled to a consumable including the tank and the heating element. The consumable may also be referred to as a cartomizer. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu(RTM) e-cigarette. The myblu(RTM) e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, "e-liquid" is heated by a heating device to produce an aerosol vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

In aerosol delivery devices, it is desirable to ensure that flavour aerosol can be provided consistently over the lifetime of the consumable, and that the risk of leakage is minimised.

The present invention has been devised in light of the above considerations.

### Summary of the Invention

At its most general, the present invention relates to a refill device for an aerosol delivery device.

According to a first aspect of the present invention, there is provided a refill device for an aerosol delivery device comprising:
a porous liquid transfer element containing aerosol precursor; and
an engagement unit, located at one end of the refill device and including at least a portion of the porous liquid transfer element, the engagement unit being suitable for engaging with a corresponding engagement unit of the aerosol delivery device, so as to allow aerosol precursor to flow from the porous liquid transfer element into the aerosol delivery device.

Advantageously, such a device can be used to refill, recharge, or charge an aerosol generator of the aerosol delivery device to help facilitate consistent provision of aerosol during use of the aerosol delivery device. The porous liquid transfer element may be configured to contact an aerosol generator of the aerosol delivery device when the engagement unit of the refill device is engaged with the corresponding engagement unit of the aerosol delivery device.

Optionally, the device may further comprise a liquid reservoir, fluidly connected to the porous liquid transfer element and containing aerosol precursor. The porous liquid transfer element may extend into the liquid reservoir. The liquid reservoir may contain no more than 5 ml of aerosol precursor. The refill device may further comprise a pump which, when operated may pressurise the liquid reservoir to a pressure above an ambient pressure (e.g. a pressure above atmospheric pressure).

Conveniently, the engagement unit may be a cavity into which a mouthpiece of the aerosol delivery device can be introduced, and a portion of the porous liquid transfer element may extend into the cavity so as to contact a protruding portion of the aerosol generator within the mouthpiece of the aerosol delivery device.

Optionally, the porous liquid transfer element may be at least partially contained within a housing. The engagement unit may be integrally formed from the housing. When the refill device includes a liquid reservoir, the housing may be deformable so as to exert a pressure on aerosol precursor stored within the liquid reservoir when a force is applied to the housing.

In a second aspect, the invention provides a reservoir-less aerosol delivery device usable with the refill device of the first aspect, the reservoir-less aerosol delivery device comprising:
an aerosol generator, configured to generate an aerosol from an aerosol precursor; and
a mouthpiece, in which a portion of the aerosol generator is provided;
wherein the aerosol generator is not provided with a reservoir of aerosol precursor, and the aerosol generator is charged with aerosol precursor solely through use of the refill device.

By not provided with a reservoir of aerosol precursor, it may be meant that the reservoir-less aerosol delivery device does not comprise a reservoir which is distinct from the aerosol generator and which is fluidly connected to the aerosol generator.

In a third aspect, the invention provides a method of charging an aerosol generator of an aerosol delivery device using the refill device according to the first aspect, the method comprising the step of:
engaging the engaging unit of the refill device with the corresponding engagement unit of the aerosol deliver device, such that aerosol precursor flows form the porous liquid transfer element into the aerosol delivery device.

The refill device as used in the third aspect may have any, or any combination insofar as they are compatible, of the optional features of the first aspect.

When the refill device includes a pump, the method may include a step of actuating the pump after the engaging unit of the refill device has engaged the corresponding engagement unit of the aerosol delivery device.

In a fourth aspect, the invention provides a smoking substitute system, including an aerosol delivery device and the refill device of the first aspect (including any, or any combination insofar as they are compatible, of the optional features disclosed therein), wherein the aerosol delivery device includes an aerosol generator connected to the porous liquid transfer element and a corresponding engagement unit engaged with the engagement unit of the refill device.

The aerosol generator may include:
an aerosol generator portion configured to receive the aerosol precursor; and
an airflow passage, configured to direct air past the aerosol generator portion to pick up the aerosol precursor from the aerosol generator portion to form an aerosol.

The aerosol generator portion may include a Venturi aperture and a porous member located within the Venturi aperture.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Fig. 1** shows a schematic drawing of a smoking substitute device;
**Fig. 2** shows a schematic drawing of a smoking substitute device;
**Fig. 3** shows a schematic drawing of a smoking substitute device;
**Fig. 4** shows a schematic drawing of a smoking substitute device;
**Fig. 5** shows a cross-sectional view of a consumable in a deactivated state;
**Fig. 6** shows a cross-sectional view of the consumable of Fig. 5 in an activated state;
**Fig. 7** a cross-sectional view of a flavour pod portion of a consumable;
**Fig. 8a** shows a top view of a flavour pod portion of a consumable;
**Fig. 8b** shows a cut away perspective view of a flour pod portion of a consumable;
**Fig. 9** shows a refill device engaged with an aerosol delivery device; and
**Fig. 10** shows a variant refill device for an aerosol delivery device.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

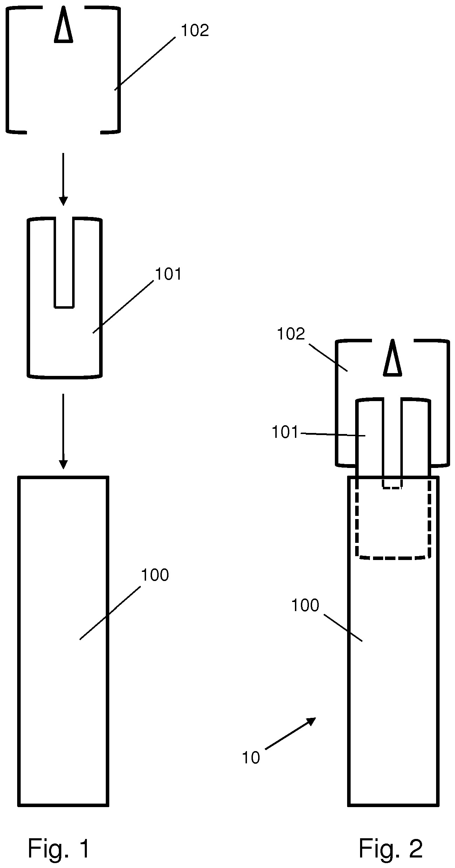

Referring to Figures 1 and 2, there is shown a smoking substitute device 10. In this example, the smoking substitute device comprises a cartomiser 101 and a flavour pod 102 connected to a base unit 100. In this example, the base unit 100 includes elements of the smoking substitute device such as a battery, an electronic controller, and a pressure transducer. The cartomiser 101 may engage with the base unit 100 via a push-fit engagement, a screw-thread engagement, or a bayonet fit, for example. A cartomiser may also be referred to as a "pod". The smoking substitute device can include an aerosol delivery device according to the present invention.

The flavour pod 102 is configured to engage with the cartomiser 101 and thus with the base unit 100. The flavour pod 102 may engage with the cartomiser 101 via a push-fit engagement, a screw-thread engagement, or a bayonet fit, for example. Fig. 2 illustrates the cartomiser 101 engaged with the base unit 100, and the flavour pod 102 engaged with the cartomiser 101. As will be appreciated, in this example, the cartomiser 101 and the flavour pod 102 are distinct elements. Each of the cartomiser 101 and the flavour pod may be an aerosol delivery device according to the present invention.

As will be appreciated from the following description, the cartomiser 101 and the flavour pod 102 may alternatively be combined into a single component that implements the functionality of the cartomiser 101 10 and flavour pod 102. Such a single component may also be an aerosol delivery device according to the present invention. In other examples, the cartomiser may be absent, with only a flavour pod 102 present.

A "consumable" component may mean that the component is intended to be used once until exhausted, and then disposed of as waste or returned to a manufacturer for reprocessing.

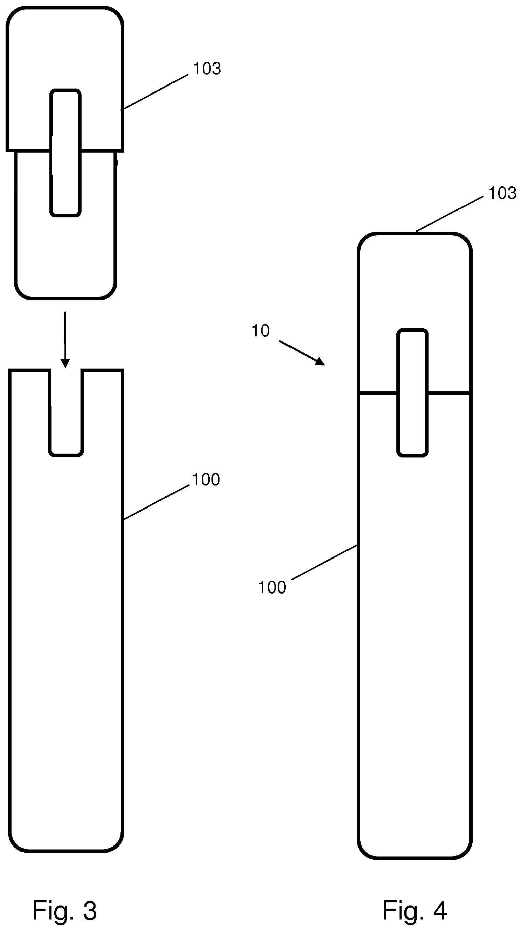

Referring to Figures 3 and 4, there is shown a smoking substitute device comprising a base unit 100 and a consumable 103. The consumable 103 combines the functionality of the cartomiser 101 and the flavour pod 102. In Figure 3, the consumable 103 and the base unit 100 are shown separated from one another. In Figure 4, the consumable 103 and the base unit 100 are engaged with each other to form the smoking substitute device 10.

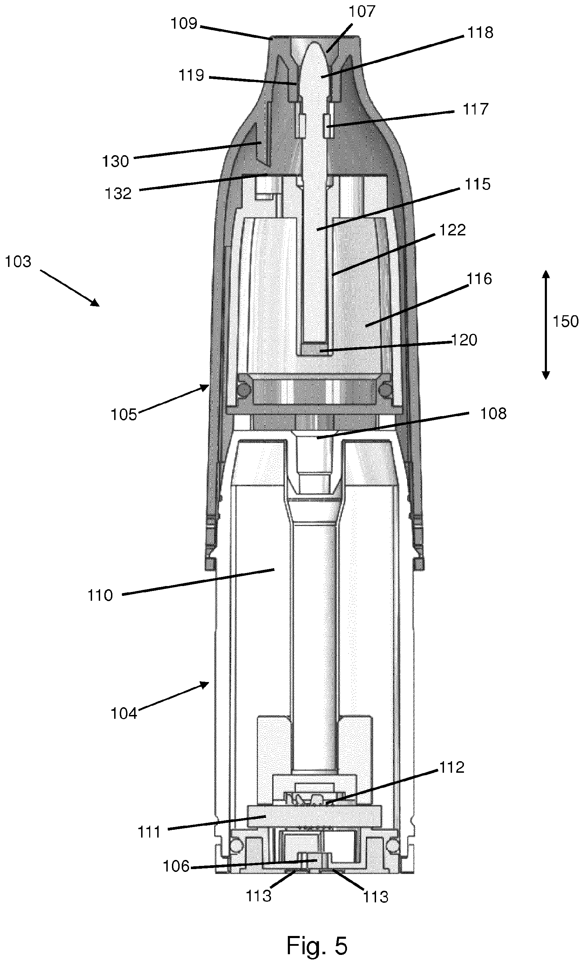

Referring to Figure 5, there is shown a consumable 103 engagable with a base unit via a push-fit engagement in a deactivated state. The consumable 103 may be considered to have two portions - a cartomiser portion 104 and a flavour pod portion 105, both of which are located within a single component (as in Figures 3 and 4).

The consumable 103 includes an upstream airflow inlet 106 and a downstream airflow outlet 107. In other examples a plurality of inlets and/or outlets are included. Between and fluidly connecting the inlet 106 and the outlet 107 there is an airflow passage 108. The outlet 107 is located at the mouthpiece 109 of the consumable 103, and is formed by a mouthpiece aperture.

As above, the consumable 103 includes a flavour pod portion 105. The flavour pod portion 105 is configured to generate a first (flavour) aerosol for output from the outlet 107 of the mouthpiece 109 of the consumable 103. The flavour pod portion 105 of the consumable 103 includes a member 115. The member 115 acts as a passive aerosol generator (e.g. an aerosol generator which does not use heat to form the aerosol, also referred to as a "first aerosol generator" in this example), and is formed of a porous material. The member 115 comprises a supporting portion 117, which is located inside a housing, and an aerosol generator portion 118, which is located in the airflow passage 108. In this example, the aerosol generator portion 118 is a porous nib.

When activated, as discussed in more detail below, a first storage 116 (in this example a tank) for storing a first aerosol precursor (i.e. a flavour liquid) is fluidly connected to the member 115. The porous nature of the member 115 means that flavour liquid from the first storage 116 is drawn into the member 115. As the first aerosol precursor in the member 115 is depleted in use, further flavour liquid is drawn from the first storage 116 into the member 115 via a wicking action. Before activation, the first storage 116 is fluidly isolated from the member 115. In this example, the isolation is achieved via plug 120 (preferably formed from silicon) located at one end of a conduit 122 containing the member 115. In other examples, the plug may be replaced by any one of: a duck bill valve; a split valve or diaphragm; or a sheet of foil. The first storage 116 further includes a pressure relief opening 132, which in the deactivated state is sealed by a pierceable cover (preferably made from foil). Piercing member 130, which is formed as a part of the mouthpiece 109 and may take the form of a blade, pierces the pierceable cover and opens the pressure relief opening 132 when the consumable is moved to the activated state (as is discussed in more detail below).

As described above, the aerosol generator portion 118 is located within the airflow passage 108 through the consumable 103. The aerosol generator portion 118 therefore constricts or narrows the airflow passage 108. The aerosol generator portion 118 occupies some of the area of the airflow passage, resulting in constriction of the airflow passage 108. The airflow passage 108 is narrowest adjacent to the aerosol generator portion 118. Since the constriction results in increased air velocity and corresponding reduction in air pressure at the aerosol generator portion 118, the constriction is a Venturi aperture 119. The constriction is generally toroidal in shape, and may include one or more intersections where supports contact the aerosol generator portion 118.

The cartomiser portion 104 of the consumable 103 includes a second storage 110 (in this example a tank) for storing a second aerosol precursor (i.e. e-liquid, which may contain nicotine). Extending into the second storage 110 is a wick 111. The wick 111 is formed from a porous wicking material (e.g. a polymer) that draws second aerosol precursor from the second storage 110 into a central region of the wick 111 that is located outside the e-liquid storage tank 110.

A heater 112 is a configured to heat the central region of the wick 111. The heater 112 includes a resistive heating filament that is coiled around the central region of the wick 111. The wick 111, the heater 112 and the e-liquid storage tank 110 together act as an active aerosol generator (i.e. an aerosol generator which uses heat to form the aerosol, referred to as a "second aerosol generator" in this example).

As described above, the first and second aerosol generators are both at least partially located within the airflow passage 108, with the first aerosol generator downstream (with respect to air flow in use) of the second aerosol generator.

So that the consumable 103 may be supplied with electrical power for activation of the heater 112, the consumable 103 includes a pair of consumable electrical contacts 113. The consumable electrical contacts 113 are configured for electrical connection to a corresponding pair of electrical supply contacts in the base unit 100. The consumable electrical contacts 113 are electrically connected to the electrical supply contacts 114 when the consumable 103 is engaged with the base unit 100. The base unit 100 includes an electrical power source (not shown), for example a battery.

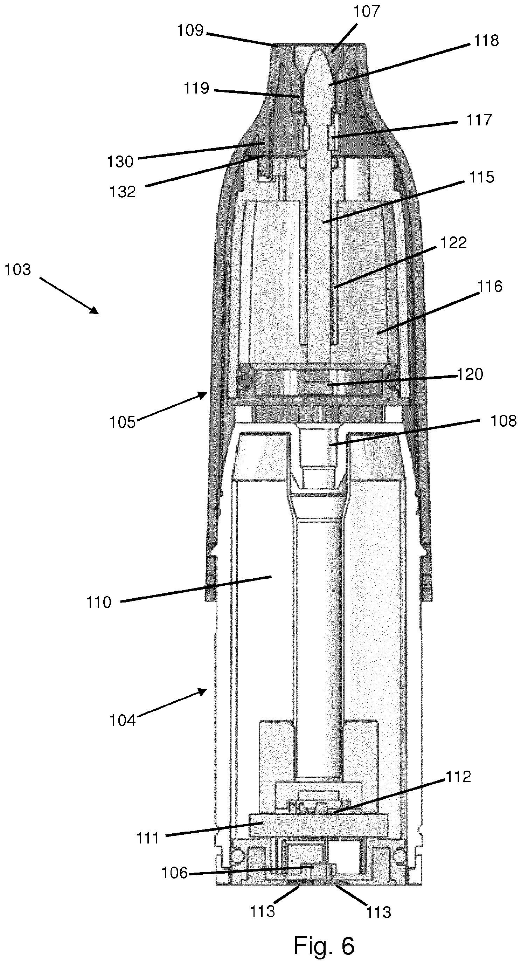

Figure 6 shows the consumable 103 of Figure 5 in an activated state, like features are indicated by like reference numerals. To transition from the deactivated state to the activated state, mouthpiece 109 is moved along a central axis 150 towards cartomizer portion 104 (e.g. one along which the consumable extends, and along which member 115 extends). The mouthpiece 109, via supporting portion 117, is fixed to the member 115 and therefore member 115 moves with the mouthpiece 109. The mouthpiece 109, and member 115, is moved relative to the tank 116. Piercing member 130 therefore contacts, and pierces, pressure relief opening 132 thereby fluidly connecting the airflow passage 108 to an interior of the first storage 116. Further, member 115 pushes on, and moves, plug 120 out of the conduit 122 which then allows member 115 to fluidly connect with the first aerosol precursor stored in the first storage 116. The plug 120 may then be unconstrained within the first storage, or may be pushed by member 115 into a holding location.

Once activated, and in use, a user draws (or "sucks", "pulls", or "puffs") on the mouthpiece 109 of the consumable 103, which causes a drop in air pressure at the outlet 107, thereby generating airflow through the inlet 106, along the airflow passage 108, out of the outlet 107 and into the user's mouth.

When the heater 112 is activated (by passing an electric current through the heating filament in response to the user drawing on the mouthpiece 109, the drawing of air may be detected by a pressure transducer) the e-liquid located in the wick 111 adjacent to the heating filament is heated and vaporised to form a vapour. The vapour condenses to form the second aerosol within the airflow passage 108. Accordingly, the second aerosol is entrained in an airflow along the airflow flow passage 108 to the outlet 107 and ultimately out from the mouthpiece 109 for inhalation by the user when the user 10 draws on the mouthpiece 109.

The base unit 100 supplies electrical current to the consumable electrical contacts 113. This causes an electric current flow through the heating filament of the heater 112 and the heating filament heats up. As described, the heating of the heating filament causes vaporisation of the e-liquid in the wick 111 to form the second aerosol.

As the air flows up through the airflow passage 108, it encounters the aerosol generator portion 118. The constriction of the airflow passage 108 caused by the aerosol generator portion 118 results in an increase in air velocity and corresponding decrease in air pressure in the airflow in the vicinity of the porous surface 118 of the aerosol generator portion 115. The corresponding low pressure and high air velocity region causes the generation of the first (flavour) aerosol from the porous surface 118 of the aerosol generator portion 118. The first (flavour) aerosol is entrained into the airflow and ultimately is output from the outlet 107 of the consumable 103 and thus from the mouthpiece 109 into the user's mouth.

The first aerosol is sized to inhibit pulmonary penetration. The first aerosol is formed of particles with a mass median aerodynamic diameter that is greater than or equal to 15 microns, in particular, greater than 30 microns, more particularly greater than 50 microns, yet more particularly greater than 60 microns, and even more particularly greater than 70 microns.

The first aerosol is sized for transmission within at least one of a mammalian oral cavity and a mammalian nasal cavity. The first aerosol is formed by particles having a maximum mass median aerodynamic diameter that is less than 300 microns, in particular less than 200 microns, yet more particularly less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

As a brief aside, it will be appreciated that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

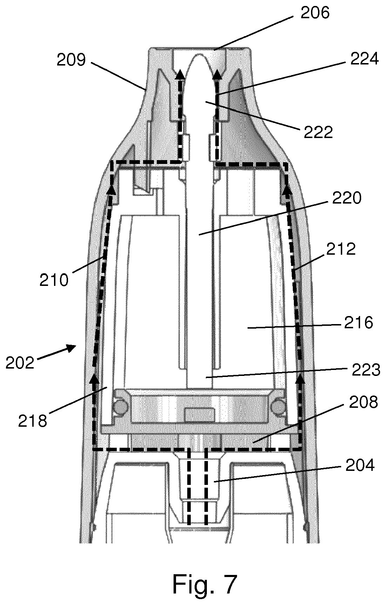

Referring to Fig. 7, there is shown a flavour pod portion 202 of a consumable in an activated state, the consumable providing an aerosol delivery device in accordance with the invention. The consumable further comprises a cartomiser portion (not shown in Fig. 7) having all of the features of the cartomiser portion 104 described above with respect to Figs. 5 and 6. However, in other examples, the consumable does not comprise the cartomiser portion, and provides only flavour to the user.

The flavour pod portion 202 comprises an upstream (i.e. upstream with respect to flow of air in use) inlet 204 and a downstream (i.e. downstream with respect to flow of air in use) outlet 206. Between and fluidly connecting the inlet 204 and the outlet 206 the flavour pod portion 204 comprises an airflow passage 208. The airflow passage 208 comprises a first airflow branch 210 and a second airflow branch 212, each of the first airflow branch 210 and the second airflow branch 212 fluidly connecting the inlet 204 and the outlet 206. In other examples the airflow passage 208 may have an annular shape. The outlet 206 is located at the mouthpiece 209 of the consumable 103, and is also referred to as a mouthpiece aperture 206.

The flavour pod portion 202 comprises a storage 214, which stores a first aerosol precursor. The storage 214 comprises a reservoir 216 located within a chamber 218. The reservoir 216 is formed of a first porous material.

The flavour pod portion 202 comprises a member 220, which comprises an aerosol generator portion 222 and a supporting portion 223. The aerosol generator portion 222 is located at a downstream end (an upper end in Fig. 6) of the member 220, while the supporting portion 223 makes up the rest of the member 220. The supporting portion 223 is elongate and substantially cylindrical. The aerosol generator portion 222 is bulb-shaped, and comprises a portion which is wider than the supporting portion 223. The aerosol generator portion 222 tapers to a tip at a downstream end of the aerosol generator portion 222.

The member 220 extends into and through the storage 214. The member 220 is in contact with the reservoir 216. More specifically, the supporting portion 223 extends into and through the storage 204 and is in contact with the reservoir 216. The member 220 is located in a substantially central position within the reservoir 216 and is substantially parallel to a central axis of the consumable. The member 220 is formed of a second porous material.

The first and second airflow branches 210, 212 are located on opposite sides of the member 220. Additionally, the first and second airflow branches 210, 212 are located on opposite sides of the reservoir 216. The first and second airflow branches 210, 212 branch in a radial outward direction (with respect to the central axis of the consumable 200) downstream of the inlet 204 to reach the opposite sides of the reservoir 216.

The aerosol generator portion 222 is located in the airflow passage 208 downstream of the first and second airflow branches 210, 212. The first and second airflow branches 210, 212 turn in a radially inward direction to merge at the member 220, at a point upstream of the aerosol generator portion 222.

The aerosol generator portion 222 is located in a narrowing section 224 of the airflow passage 208. The narrowing section 224 is downstream of the point at which the first and second airflow branches 210 212 merge, but upstream of the mouthpiece aperture 207. The mouthpiece aperture 207 flares outwardly in the downstream direction, such that a width of the mouthpiece aperture 207 increases in the downstream direction.

In use, when a user draws on the mouthpiece 209, air flow is generated through the air flow passage 208. Air (comprising the second aerosol from the cartomiser portion as explained above with respect to Fig. 5) flows through the inlet 204 before the air flow splits to flow through the first and second airflow branches 210, 212. Further downstream, the first and second airflow branches 210, 212 provide inward airflow towards the member 220 and the aerosol generator portion 222.

As air flows past the aerosol generator portion in the narrowing section 224, the velocity of the air increases, resulting in a drop in air pressure. This means that the air picks up the first aerosol precursor from the aerosol generator portion 222 to form the first aerosol. The first aerosol has the particle size and other properties described above with respect to Fig. 5.

As the first aerosol precursor is picked up by the air, the member 220 transfers further first aerosol precursor from the storage 214 to the aerosol generator portion 222. More specifically, the member 220 wicks the first aerosol precursor from the storage 214 to the aerosol generator portion 224.

In other examples, the storage 214 comprises a tank containing the first aerosol precursor as free liquid, rather than the reservoir 216 and the chamber 218. In such examples, the member 220 still extends into the tank to transfer first aerosol precursor from the tank to the aerosol generator portion 224.

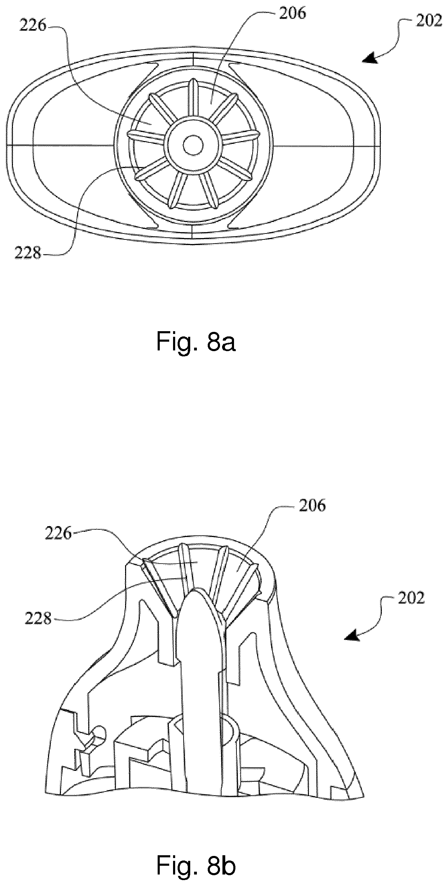

Figs. 8a and 8b show further views of the flavour pod portion 202 which highlight features of the mouthpiece 209. Many of the reference numerals of Fig. 7 are omitted from Fig. 8a and 8b for clarity.

The mouthpiece aperture 206 comprises an inner surface 226, which is uneven. In the present example, the inner surface 226 has the form of a substantially frustoconical surface, but includes grooves or channels 228 to make the inner surface 226 somewhat uneven. In other examples, the inner surface 226 may have another form (for example, the form a substantially cylindrical surface), and may include any type of protrusion or groove to make the inner surface uneven.

The inner surface 226 is angled with respect to an axial direction (i.e. relative to a central axis extending from a base of the consumable to the mouthpiece) such that the width of the mouthpiece aperture 209 increases in the downstream direction. The inner surface 226 is immediately downstream of the narrowing section 224 of the airflow passage 108.

The grooves 228 are generally v-shaped in cross-sectional profile, and extend in the axial direction for the full length of the inner surface 226. Each groove 228 is formed from a pair of surfaces angled at between 30 and 90 degrees relative to each other. More specifically, each groove 228 is formed from a pair of surfaces angled at 60 degrees relative to each other.

The grooves 228 have a depth (measured normal to the inner surface 226) of at least 0.2 mm. More specifically, the grooves 228 have a depth of at least 0.3 mm. More specifically, the grooves 228 have a depth of at least 0.4 mm.

The grooves 228 have a depth of less than 0.8 mm. More specifically, the grooves have a depth of less than 0.7 mm. More specifically, the grooves have a depth of less than 0.6 mm.

More specifically, the grooves have a depth of substantially 0.5 mm.

The grooves 228 are substantially equi-spaced in a circumferential manner around the inner surface 226. The inner surface 226 comprises at least 6 grooves. More specifically, the inner surface comprises at least 7 grooves. More specifically, the inner surface 226 comprises at least 8 grooves.

The inner surface 226 comprises at most 12 grooves 228. More specifically, the inner surface 226 comprises at most 11 grooves 228. More specifically, the inner surface 226 comprises at most 10 grooves 228.

More specifically, the inner surface 226 comprises 9 grooves 228.

The grooves 228 are spaced apart from each other by substantially 1 mm at the downstream end of the inner surface 226. In other examples, the spacing at the downstream end of grooves or protrusions may be selected such that it is equal to or less than the mass median diameter (as described above) of particles in the first aerosol.

The inner surface 226 comprises a smooth polished surface between the grooves 228. Polishing the surface in this way provides improved aerodynamic properties. However, in other examples, the inner surface 226 may be textured. In such examples, the texture of the surface may provide the uneven surface, and no grooves are required,

In use, the uneven nature of the inner surface 226 makes it easier for droplets to form on the inner surface 226, preventing large droplets from entering the user's mouth. The grooves 228 help to channel the large droplets back into the consumable.

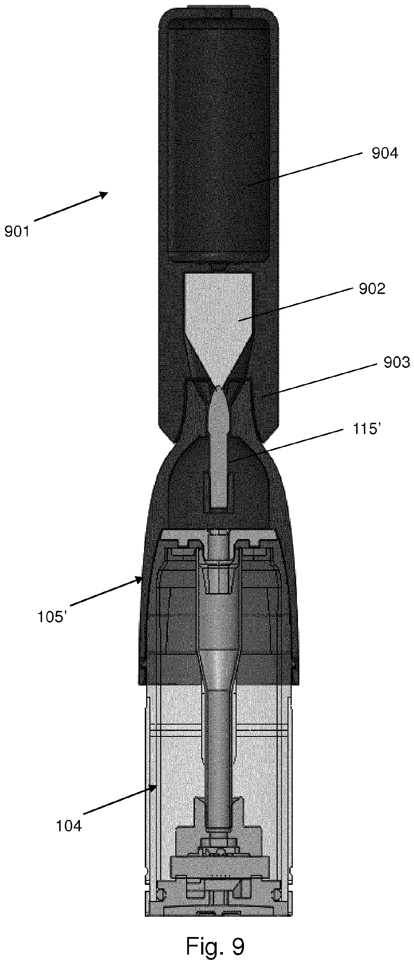

Figure 9 shows a refill device 901 for use with the consumable discussed previously. The refill device includes: a porous liquid transfer element 902 which contains first aerosol precursor, located towards one end of the device; an engagement unit 903, adjacent the porous liquid transfer element; and a liquid store 904, which contains further first aerosol precursor. The liquid store 904 and porous liquid transfer element 902 are within an outer housing, a portion of which provides the engagement unit 903. The housing, liquid store, and engagement unit in this example are formed from plastic. The porous liquid transfer element 902 is formed of a fibrous material.

The engagement unit 903 provides a cavity which has a corresponding shape to mouthpiece 109 of the flavour pod portion 105. In this example, the cavity is frustoconical in shape, and has a lip which aligns with an uppermost lip of the mouthpiece 109. This can ensure that the refill device is not pressed to far onto the mouthpiece 109, and so the risk of damaging the member 115 is minimised.

In use, the refill device 901 is engaged with the mouthpiece of the flavour pod portion 105 discussed above. First aerosol precursor then leaches from the porous liquid transfer element 902 into the member 115. This can be useful if, for example, the first store 116 is deleted of first aerosol precursor. The refill device can then be used to refill, or charge, the member 115 so that it may still function as an aerosol generator.

Alternatively, as shown in Figure 9, a variant flavour pod portion 105' may be provided. Variant flavour pod portion 105' is attached to the same cartomiser portion 104, but has a shorter variant member 115' than the examples shown previously. Further, the variant flavour pod portion 105' contains no first reservoir. Therefore the variant member 115' must be charged using the refill device before at least the first use of the flavour pod portion 105'. Advantageously, such a variant flavour pod portion 105' requires fewer parts, has smaller dimensions, and is impervious to leakage during transit (as no first aerosol precursor is present until the first time refill device 901 is used to charge variant member 115'. As before, the variant member 115' includes an aerosol generating portion located with the mouthpiece 109 of the flavour pod 105'.

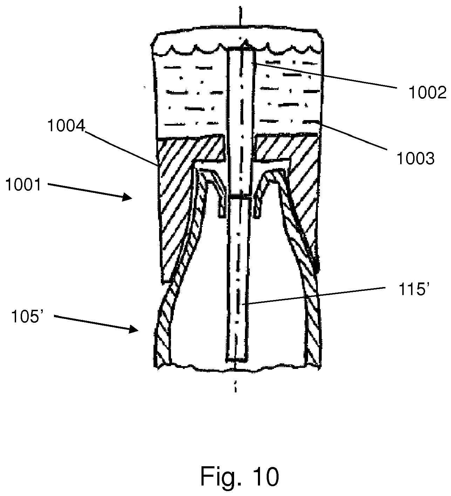

Figure 10 shows a variant refill device 1001, engaged to the variant flavour pod 105' discussed previously. Variant refill device 1001 differs from refill device 901 in that the porous liquid transfer element 1002 extends into the liquid reservoir 1002 (whereas, in refill device 901, the porous liquid transfer element merely abutted an aperture in liquid reservoir 903). Advantageously, this can promote the transfer of first aerosol precursor stored in the liquid reservoir 1002 into the porous liquid transfer element 1002. Variant refill device 1001 also contains an engagement unit 1004, which is configured in a similar manner to engagement unit 104 discussed above.

In both refill devices (as shown in Figures 9 and 10), the refill device has an engagement portion or unit which is corresponding or complementary in shape to an engagement unit of the flavour pod 105'. In these examples, the engagement unit of the flavour pod 105' is the mouthpiece, and so the engagement portion or unit of the refill device has a cavity corresponding in shape to the mouthpiece.

The housing forming the refill device 901 or variant refill device 1001 may be deformable. Therefore, when a user squeezes or otherwise applies a force to the housing this force may be communicated to the liquid reservoir so as to impart a pressure to the aerosol precursor stored therein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A refill device for an aerosol delivery device comprising:
a porous liquid transfer element containing aerosol precursor; and
an engagement unit, located at one end of the refill device and including at least a portion of the porous liquid transfer element, the engagement unit being suitable for engaging with a corresponding engagement unit of the aerosol delivery device, so as to allow aerosol precursor to flow from the porous liquid transfer element into the aerosol delivery device.

2. The refill device of claim 1, further comprising a liquid reservoir, fluidly connected to the porous liquid transfer element and containing aerosol precursor.

3. The refill device of claim 2, wherein the porous liquid transfer element extends into the liquid reservoir

4. The refill device of either of claims 2 or 3, wherein the liquid reservoir contains no more than 5 ml of aerosol precursor.

5. The refill device of any of claims 2 to 4, further comprising a pump which, when operated, pressurises the liquid reservoir to a pressure above an ambient pressure.

6. The refill device of any preceding claim, wherein the engagement unit is a cavity, into which a mouthpiece of the aerosol delivery device can be introduced, and wherein a portion of the porous liquid transfer element extends into the cavity so as to contact a protruding portion of an aerosol generator within the mouthpiece of the aerosol delivery device.

7. The refill device of any preceding claim, wherein the porous liquid transfer element is partially contained within a housing.

8. The refill device of claim 7, wherein the engagement unit is integrally formed from the housing.

9. The refill device of any of claims 7 - 8 as dependent on claim 2, wherein the housing is deformable so as to exert a pressure on aerosol precursor stored within the liquid reservoir when a force is applied to the housing.

10. A reservoir-less aerosol delivery device usable with the refill device of any preceding claim, the reservoir-less aerosol delivery device comprising:
an aerosol generator, configured to generate an aerosol from an aerosol precursor; and
a mouthpiece, in which a portion of the aerosol generator is provided;
wherein the aerosol generator is not provided with a reservoir of aerosol precursor, and the aerosol generator is charged with aerosol precursor solely through use with the refill device.

11. A method of charging an aerosol generator of an aerosol delivery device using the refill device of any of claims 1 - 9, comprising:
engaging the engaging unit of the refill device with the corresponding engagement unit of the aerosol delivery device, such that aerosol precursor flows from the porous liquid transfer element into the aerosol delivery device.

12. The method of claim 11 as dependent on claim 5, further comprising a step of actuating the pump after the engaging unit of the refill device has engaged the corresponding engagement unit of the aerosol delivery device.

13. A smoking substitute system, including an aerosol delivery device and the refill device of any of claims 1 - 9, wherein the aerosol delivery device includes an aerosol generator connected to the porous liquid transfer element and a corresponding engagement unit engaged with the engagement unit of the refill device.

14. The smoking substitute system of claim 13, wherein the aerosol generator includes:
an aerosol generator portion configured to receive the aerosol precursor; and
an airflow passage, configured to direct air past the aerosol generator portion to pick up the aerosol precursor from the aerosol generator portion to form an aerosol.

15. The smoking substitute system of claim 14, wherein the aerosol generator portion includes a Venturi aperture and a porous member located within the Venturi aperture.
